# EUROPEAN PATENT APPLICATION

(11) **EP 3 366 776 A1**
(43) Date of publication of application: **29.08.2018**
(21) Application number: 16857824.3
(22) Date of filing: 21.10.2016
(51) Int. Cl.: C12N 15/70, C12N 15/77, C12N 9/10, C12N 9/88

(54) **RECOMBINANT MICROORGANISM FOR PRODUCING L-THREONINE AND METHOD FOR PRODUCING L-THREONINE BY USING SAME**

(30) Priority: 23.10.2015 KR 20150148004
(71) Applicant: CJ Cheiljedang Corporation, Seoul 04560 (KR)
(72) Inventor: SEO, Chang Il, Incheon 21325 (KR); PARK, Hye Min, Suwon-si Gyeonggi-do 16508 (KR); LEE, Kwang Ho, Daejeon 34140 (KR); PARK, Sang Min, Suwon-si Gyeonggi-do 16236 (KR); KIM, Kyung Rim, Seoul 04425 (KR); CHEONG, Ki Yong, Suwon-si Gyeonggi-do 16708 (KR)
(74) Representative: Nony
(86) International application number: PCT/KR2016/011907
(87) International publication number: WO 2017/069567

(57) **Abstract**

The present disclosure relates to a recombinant microorganism producing threonine, and a method for producing L-threonine using the same.

## Description

### [Technical Field]

The present disclosure relates to a recombinant microorganism producing threonine, and a method for producing L-threonine using the same.

### [Background Art]

Threonine, an essential amino acid, is widely used in feeds, food additives, and animal growth promoters, and is also effectively used in rehydration solutions and synthetic materials for medical and pharmaceutical uses.

With regard to a method for producing threonine using a microorganism, methods for enhancing threonine biosynthesis genes (*e.g., ppc, aspC,* and *thrABC*) and for blocking a threonine degradation pathway are known for increasing the yield (Kwang-Ho Lee, et al., Molecular System Biology 2007). The genes involved in the threonine degradation pathway include *tdh, tdcB, glyA, ilvA, etc.,* and among these, threonine deaminase (*ilvA*) is known to be the most important threonine degradation gene. The deletion of *ilvA* among degradation genes greatly improves the yield of threonine, but is problematic in that an expensive isoleucine auxotroph appears; therefore, it is generally known to apply attenuation of the *ilvA* activity and high-susceptibility mutation for isoleucine (Akhverdian Valery Z, *et al.*).

Meanwhile, a strain of the genus *Corynebacterium* or a strain of the genus *Escherichia* produces 2-ketobutyrate and biosynthesizes isoleucine through ilvA, which is the gene involved in the threonine degradation pathway. However, it is known that a microorganism such as *Methanococcus jannaschii* can synthesize 2-ketobutyrate and isoleucine using acetyl-CoA and pyruvate as substrates through citramalate synthase (cimA) (Atsumi, Liao JC, Appl Environ Microbiol., 2008).

### [Disclosure]

### [Technical Problem]

The present inventors have attempted to develop a method for effectively producing threonine. As a result, they have confirmed that the yield of threonine production was improved by a method of reducing the activity of the threonine deaminase (IlvA) enzyme in the threonine-producing strain, and by a method of introducing the gene of citramalate synthase (cimA), and have also confirmed that the existing problem (Xie X, et al., J Ind Microbiol Biotechnol. 2014), wherein the culture is delayed due to accumulation of acetate, could be overcome, thereby completing the present disclosure.

### [Technical Solution]

An object of the present disclosure is to provide a recombinant microorganism that produces threonine with high efficiency.

Another object of the present disclosure is to provide a method for producing threonine using the microorganism above.

### [Advantageous Effects]

The recombinant microorganism of the present disclosure, which produces threonine, maintains or improves its growth ability and has excellent threonine productivity because the amount of acetate accumulation in the microorganism is reduced by reducing or inactivating the activity of threonine deaminase (ilvA) and by introducing the activity of citramalate synthase (cimA). Accordingly, the microorganism can be widely used for the efficient and economical production of threonine even on an industrial scale.

### [Brief Description of Drawing]

Fig. 1 is a schematic diagram showing a gene map of a *pCJ-MuAB* plasmid, which is a helper plasmid used in a mini-Mu gene insertion method.
Fig. 2 is a schematic diagram showing a gene map of a *pMu-R6K* plasmid, which is an integrative plasmid used in a mini-Mu gene insertion method that is used for introduction of a threonine biosynthesis gene.

### [Best Mode]

In order to achieve the objects above, an aspect of the present disclosure provides a recombinant microorganism producing threonine, wherein an activity of threonine deaminase (IlvA) is reduced or inactivated and an activity of citramalate synthase is retained.

As used herein, the term "threonine" refers to an amino acid with the formula HO₂CCH(NH₂)CH(OH)CH₃, which is a hydroxy-α-amino acid, one of the essential amino acids that are not produced in the body. In the present disclosure, threonine may be an L- or D-type optical isomer, but is not limited thereto.

As used herein, the term "threonine productivity" refers to an ability of a microorganism to produce and accumulate threonine in the microorganism or in the medium in which the microorganism is cultured. Such threonine productivity may be a property retained by a wild-type strain or a property imparted or enhanced by modification of the strain.

As used herein, the term "threonine deaminase (EC4.3.1.19)" may be referred to as threonine ammonia-lyase or threonine dehydratase, and is an enzyme catalyzing a reaction of converting threonine to α-ketobutyrate and ammonia. The α-ketobutyrate produced therefrom is used as a precursor of isoleucine. Therefore, when the threonine deaminase is deficient, the biosynthesis of isoleucine becomes abnormal, and thus the growth of microorganisms may be retarded.

As used herein, the terms "threonine deaminase enzyme" and "gene encoding the same" may include any proteins having the activity of threonine deaminase and any genes encoding the same, but these are not limited thereto.

Specifically, the threonine deaminase may be easily obtained from a known database such as the National Center for Biotechnology Information (NCBI) or the DNA Data Bank of Japan (DDBJ), and for example, it may be represented by the amino acid sequence of SEQ ID NO: 7. However, because the amino acid sequence of the protein showing the activity may differ depending on species or strain of the microorganism, the protein is not limited thereto.

Additionally, if the protein is a protein substantially having the activity of threonine deaminase, and if the protein is composed of an amino acid sequence having 70% or more homology to an amino acid sequence of SEQ ID NO: 7, specifically 80% or more homology thereto, more specifically 90% or more homology thereto, much more specifically 95% or more homology thereto, much more specifically 98% or more homology thereto, and most specifically 99% or more homology thereto, the protein can be included in the scope of the present disclosure without limitation.

Further, it is apparent that an amino acid sequence having homology to the sequence above, part of which is deleted, modified, substituted, or added, is also within the scope of the present disclosure, as long as the resulting amino acid sequence has a biological activity substantially equivalent or corresponding to the protein of SEQ ID NO: 7.

Additionally, the gene encoding the threonine deaminase may have a nucleotide sequence encoding the amino acid sequence of SEQ ID NO: 7. In the polynucleotide, various modifications may be made in the coding region without changing an amino acid sequence of the protein, due to codon degeneracy or in consideration of the codons preferred in an organism in which the protein is to be expressed. The polynucleotide sequence may have, for example, a polynucleotide sequence of SEQ ID NO: 30 and may have a nucleotide sequence having 80% homology thereto, specifically 90% homology thereto, and more specifically 99% homology thereto, but is not limited thereto.

As used herein, the term "homology" refers to a degree of matching with a given amino acid sequence or nucleotide sequence, and the homology may be expressed as a percentage. In the present disclosure, a homology sequence having an activity which is identical or similar to the given amino acid sequence or nucleotide sequence is expressed as "% homology". For example, the % homology may be confirmed using standard software, i.e., BLAST 2.0, for calculating parameters such as score, identity, and similarity, or by comparing sequences via southern hybridization experiments, and the appropriate hybridization condition to be defined may be determined by a method known to those skilled in the art (*e.g*., Sambrook et al., Molecular Cloning: A Laboratory Manual 1989).

As used herein, the term "reduction of enzyme activity" means that the activity of the relevant enzyme is reduced relative to an endogenous activity of the wild-type or state before modification.

Additionally, as used herein, the term "inactivation" means that the gene encoding the relevant enzyme is expressed at a low level or is not expressed, compared to that of a wild-type strain, or the activity is eliminated or reduced, even though the gene is expressed. Reduction of inactivation of the enzyme activity may be accomplished by any methods known in the art.

Specifically, the inactivation may be carried out by deletion of a part or the entirety of a polynucleotide encoding the enzyme; modification of an expression regulatory sequence for reducing expression of the polynucleotide; modification of the polynucleotide sequence on the chromosome to weaken an activity of the protein; or by a method selected from a combination thereof, but is not particularly limited thereto.

A partial or entire deletion of a polynucleotide encoding the protein may be carried out by substituting the polynucleotide encoding an endogenous target protein in the chromosome with a marker gene or a polynucleotide in which a partial nucleotide sequence was deleted, with a vector for chromosomal gene insertion.

Additionally, in order to reduce or eliminate the polynucleotide expression, an expression regulatory sequence may be modified by inducing variations in the expression regulatory sequence through deletion, insertion, conservative or non-conservative substitution of nucleotide sequence, or a combination thereof to further weaken the activity of the expression regulatory sequence, or by replacing the expression regulatory sequence with the nucleotide sequence having a weaker activity, but is not particularly limited thereto. The expression regulatory sequence may include a sequence encoding promoter, operator sequence, ribosomal binding site, and a sequence controlling the termination of transcription and translation, but is not limited thereto.

Further, modification of a polynucleotide sequence on the chromosome may be performed by inducing variations on the sequence through deletion, insertion, non-conservative or conservative substitution of the polynucleotide sequence, or a combination thereof in order to further attenuate the protein activity, or by replacing the sequence with a polynucleotide sequence which is modified to have a weaker activity, but is not limited thereto.

As used herein, the term "recombinant vector" refers to a DNA construct containing a nucleotide sequence of a polynucleotide encoding a target protein operably linked to a suitable regulatory sequence such that the target protein is expressed in a suitable host cell. The regulatory sequence may include a promoter capable of initiating transcription, any operator sequence for regulating such transcription, a sequence encoding a suitable mRNA ribosome-binding site, and a sequence for regulating the termination of transcription and translation. After being transformed into a suitable host cell, the recombinant vector may be replicated or perform its function irrespective of the host genome or may be integrated into the genome itself.

The recombinant vector used in the present disclosure is not particularly limited as long as it is replicable in a host cell, and may be produced using any vector known in the art. Examples of a commonly used vector include a natural or recombinant plasmid, a cosmid, a virus, and a bacteriophage. For example, pWE15, M13, MBL3, MBL4, IXII, ASHII, APII, t10, t11, Charon4A, and Charon21A may be used as a phage vector or a cosmid vector; and pBR-based, pUC-based, pBluescriptII-based, pGEM-based, pTZ-based, pCL-based, and pET-based may be used as a plasmid vector. The vectors that can be used in the present disclosure are not particularly limited, and expression vectors known in the art may be used. Specifically, pECCG117, pDZ, pACYC177, pACYC184, pCL, pUC19, pBR322, pMW118, and pCC1BAC vectors may be used.

For example, a polynucleotide encoding a target protein may be introduced into a chromosome through a vector for chromosomal insertion into a host cell. The introduction of the polynucleotide into a chromosome may be carried out by any method known in the art, for example, by homologous recombination, but is not limited thereto.

As used herein, the term "transformation" means that a vector containing a polynucleotide encoding a target protein is introduced into a host cell, such that the protein encoded by the polynucleotide is capable of being expressed in the host cell. The transformed polynucleotide may be any one regardless of the position as long as the polypeptide is capable of being expressed in the host cell, regardless of whether the polynucleotide is inserted and positioned into the chromosome of the host cell or positioned on an outer portion of the chromosome.

Additionally, the term "operably linked" refers to a functional connection between a promoter sequence, which initiates and mediates the transcription of the polynucleotide encoding the target protein of the present disclosure, and the gene sequence.

As used herein, the term "citramalate synthase (EC2.3.1.182)" refers to an enzyme that catalyzes the conversion of acetyl-CoA and pyruvate to citramalate and coenzyme A. This enzyme has been discovered in Archaea, such as *Methanococcus jannaschii* (Howell et al., J Bacteriol. 181: 331-333, 1999), *Leptospira interogans* (Xu et al., J Bacteriol. 186: 5400-5409, 2004), *Geobacter sulfurreducens, etc.,* and involves in the threonine-independent pathway of isoleucine biosynthesis (Risso et al., J Bacteriol. 190: 2266-2274, 2008). The enzyme is highly specific for pyruvate as a substrate and is typically inhibited by isoleucine.

As used herein, the terms "citramalate synthase enzyme" and "gene encoding the same" include, without limitation, any protein having the activity of citramalate synthase and any gene encoding the same. The sequence of the enzyme can be easily obtained from a known database such as the National Center for Biotechnology Information (NCBI) or the DNA Data Bank of Japan (DDBJ).

Specifically, the citramalate synthase enzyme may be citramalate synthase derived from *Methanococcus jannaschii* and may be represented by an amino acid sequence selected from the group consisting of SEQ ID NOS: 1, 2, and 3.

Additionally, *cimA,* which is the gene encoding the citramalate synthase, may have a nucleotide sequence encoding an amino acid sequence selected from the group consisting of SEQ ID NOS: 1, 2, and 3. Specifically, the polynucleotide sequence may have, for example, a nucleotide sequence selected from the group consisting of SEQ ID NOS: 4, 5, and 6, and may have a nucleotide sequence having 80% or more homology thereto, specifically 90% or more homology thereto, and more specifically 99% or more homology thereto, but is not limited thereto. A difference may exist in the amino acid sequence of the protein exhibiting the activity depending on the species or strain of the microorganism, or because of the degeneracy of the genetic code, a large number of functionally identical nucleic acids may encode any given protein; therefore, the polynucleotide sequence is not limited thereto.

Additionally, if the protein is a protein substantially having the activity of citramalate synthase, and if the protein is an amino acid sequence having 70% or more homology to an amino acid sequence selected from the group consisting of SEQ ID NOS: 1, 2, and 3, specifically 80% or more homology thereto, more specifically 90% or more homology thereto, much more specifically 95% or more homology thereto, much more specifically 98% or more homology thereto, and most specifically 99% or more homology thereto, the protein can be included within the scope of the present disclosure without limitation.

Further, it is apparent that an amino acid sequence having homology to the sequence above, the part of which is deleted, modified, substituted, or added, is also within the scope of the present disclosure, as long as the resulting amino acid sequence has a biological activity substantially equivalent or corresponding to the protein of the amino acid selected from the group consisting of SEQ ID NOS: 1, 2, and 3.

As used herein, the term "recombinant microorganism" may include, without limitation, a variant microorganism in which a gene is manipulated. If genetic manipulation is possible, the recombinant microorganism may be a microorganism of the genus *Escherichia,* a microorganism of the genus *Corynebacterium,* a microorganism of the genus *Brevibacterium,* a microorganism of the genus *Serratia,* or a microorganism of the genus *Providencia,* but is not limited thereto. For example, the recombinant microorganism of the present disclosure may be a microorganism of the genus *Corynebacterium* or a microorganism of the genus *Escherichia,* and specifically the microorganism of the genus *Corynebacterium* may be *Corynebacterium glutamicum,* and the microorganism of the genus *Escherichia* may be *Escherichia coli.*

In the present disclosure, the recombinant microorganism may have a parent strain having natural threonine productivity, or may have a parent strain having a variant strain in which a variation is introduced in order to enhance threonine productivity. The variation that enhances threonine productivity may be, for example, performed by enhancing genes associated with threonine biosynthesis or by attenuating genes associated with the threonine degradation pathway. The genes associated with threonine biosynthesis may be one or more genes selected from the group consisting of *pntAB* encoding pyridine nucleotide transhydrogenase, *asd* encoding aspartate-β-semialdehyde dehydrogenase, *aspC* encoding aspartate aminotransferase, *gdhA* encoding glutamate dehydrogenase, *ppc* encoding phosphoenol pyruvate carboxylase, aspA encoding aspartase, threonine operon (thrO), *etc*.; and the genes associated with the threonine degradation pathway may be one or more genes selected from the group consisting of *tdh* encoding threonine dehydrogenase, *tdcB* encoding catabolic threonine hydratase, *etc.*

Another aspect of the present disclosure provides a method for producing threonine, comprising: culturing the recombinant microorganism of the present disclosure in a medium; and recovering threonine from the microorganism or the cultured medium.

Specifically, the present disclosure provides a method for producing threonine, comprising: culturing a recombinant microorganism producing threonine, in which the activity of threonine deaminase is reduced or inactivated and the activity of citramalate synthase is introduced, in a medium; and recovering threonine from the microorganism or the cultured medium.

In the present disclosure, the recombinant microorganism producing threonine deaminase, citramalate synthase, and threonine is as described above.

As used herein, the term "culture" refers to culturing of a microorganism under artificially controlled environmental conditions. In the present disclosure, the step of culturing a microorganism, *e.g.,* a microorganism of the genus *Corynebacterium* or a microorganism of the genus *Escherichia,* may be carried out using any culture conditions and culture methods known in the art. In the method above, culturing of the microorganism of the present disclosure, *e.g*., a microorganism of the genus *Corynebacterium* or a microorganism of the genus *Escherichia,* may be carried out by, although is not particularly limited to, batch culture, continuous culture, and fed-batch culture known in the art.

The medium used for the culture must satisfy the requirements for a particular strain in an appropriate manner, and can be suitably used by those skilled in the art. The culture medium for a strain of the genus *Corynebacterium* or a strain of the genus *Escherichia* is known in the art (*e.g*., Manual of Methods for General Bacteriology. American Society for Bacteriology. Washington D.C., USA, 1981).

In particular, for the culture conditions, an appropriate pH (*e.g.,* a pH of 5 to 9, preferably a pH of 6 to 8, and most preferably a pH of 6.8) can be adjusted using a basic compound (*e.g*., sodium hydroxide, potassium hydroxide, or ammonia) or an acidic compound (*e.g*., phosphoric acid or sulfuric acid), but is not particularly limited thereto. In addition, bubble formation can be inhibited by using an anti-foaming agent, such as a fatty acid polyglycol ester.

Oxygen or an oxygen-containing gas mixture can be introduced into the culture to maintain aerobic conditions, and the temperature of the culture is usually 20°C to 45°C, preferably 25°C to 40°C. The cultivation continues until the maximum amount of threonine produced is obtained, and usually takes 10 hours to 160 hours to achieve the same. Threonine may be released into the culture medium or may be contained in the cells.

Further, the culture medium used may comprise a sugar and carbohydrate (e.g., glucose, sucrose, lactose, fructose, maltose, molasses, starch, and cellulose), oil and fat (*e.g*., soybean oil, sunflower seed oil, peanut oil, and coconut oil), fatty acid (*e.g*., palmitic acid, stearic acid, and linoleic acid), alcohol (*e.g.,* glycerol and ethanol), and organic acid (*e.g.,* acetic acid) individually or in combination as a carbon source, but the medium is not limited thereto.

In addition, the culture medium may comprise a nitrogen-containing organic compound (*e.g.,* peptone, yeast extract, meat juice, malt extract, corn solution, soybean meal powder, and urea), or an inorganic compound (*e.g*., ammonium sulfate, ammonium chloride, ammonium phosphate, ammonium carbonate, and ammonium nitrate) individually or in combination as a nitrogen source, but the medium is not limited thereto.

In addition, the culture medium may comprise potassium dihydrogen phosphate, dipotassium hydrogen phosphate, or a sodium-containing salt corresponding thereto individually or in combination as a phosphorous source, but the medium is not limited thereto.

Additionally, the culture medium may comprise other essential growth-stimulating substances including metal salts (e.g., magnesium sulfate or iron sulfate), amino acids, and vitamins, but the medium is not limited thereto.

Additionally, the culture medium may contain suitable precursors. These substances may be added to the medium during culturing in a batch or continuous manner, but are not limited thereto.

Further, recovery of threonine from the microorganism or cultured medium may be carried out by a suitable method known in the art, which is selected depending on the culture method, *e.g.,* a batch, continuous, or fed-batch culture method. Specifically, the threonine-recovering method known in the art may include centrifugation, filtration, extraction, spraying, drying, evaporation, precipitation, crystallization, electrophoresis, fractional dissolution *(e.g.,* ammonium sulfate precipitation), chromatography *(e.g.,* HPLC, ion exchange, affinity, hydrophobicity, and size exclusion), *etc.,* but is not limited thereto.

Still another aspect of the present disclosure provides the use of a recombinant microorganism, in which an activity of threonine deaminase (IlvA) is reduced or inactivated and an activity of citramalate synthase is retained, for production of threonine.

In such use, the recombinant microorganism producing threonine, in which an activity of threonine deaminase (IlvA) is reduced or inactivated and an activity of citramalate synthase is retained, is as described above.

As described above, the recombinant microorganism of the present disclosure, which produces threonine, maintains or improves its growth ability and has excellent threonine productivity because the amount of acetate accumulation in the microorganism is reduced by reducing or inactivating the activity of threonine deaminase (ilvA) and by introducing the activity of citramalate synthase (cimA).

### [Detailed Description of the Embodiment]

Hereinbelow, the present disclosure will be described in detail with accompanying exemplary embodiments. However, the exemplary embodiments disclosed herein are only for illustrative purposes and should not be construed as limiting the scope of the present disclosure.

### Example 1: Preparation of wild-type E. coli-based threonine-producing strain

### 1-1. Preparation of plasmid for gene insertion and helper plasmid

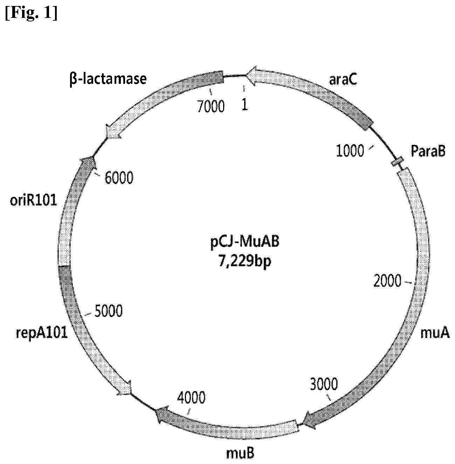

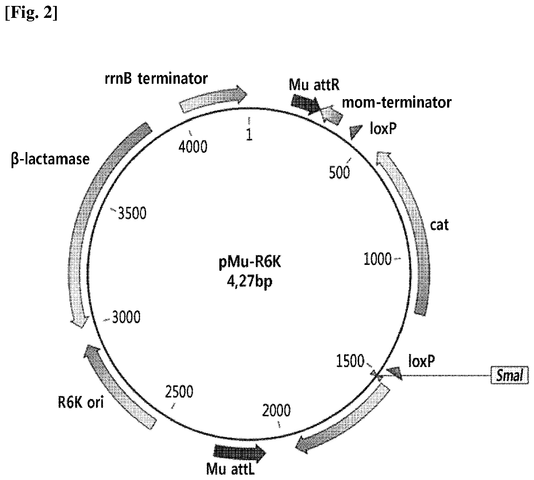

A mini-Mu transposon-based genomic insertion method (Akhverdyan VZ, Gak ER et al., Appl Microbiol Biotechnol. 2011) was used to produce threonine from wild-type *E. coli W3110* (Accession Number: ATCC9637). *pCJ-MuAB* (helper plasmid; Fig. 1) and *pMu-R6K* (integrative plasmid; Fig. 2) were prepared in order to utilize the mini-Mu insertion method. In *pCJ1-MuAB,* the helper plasmid, transposition factors MuA and MuB were expressed using an arabinose-inducible ParaB promoter. In addition, the helper plasmid was designed to easily eliminate plasmids because it contained temperature-sensitive replicons. *pMu-R6K,* the integrative plasmid, had the gene, *cat,* including Mu-attL/R, which is a mini-Mu unit, and loxP66 and loxP71 on both ends (Oleg Tolmachov, et al., Biotechnol. 2006). In addition, since the integrative plasmid had R6K replicons, the plasmid had a property of a suicidal vector, in which proliferation does not occur in normal *E. coli* without the gene *pir* (Xiao-Xing Wei, Zhen-Yu Shi.et al., Appl Microbiol Biotechnol.2010).

### 1-2. Cloning of integrative plasmid for enhancing threonine biosynthesis gene

Three integrative plasmids were prepared to enhance expression of the threonine biosynthesis gene using the plasmids prepared in Example 1-1 above.

### 1-2-1: pMu-R6K pntAB asd aspC-thrO

*pMu-R6K,* the integrative plasmid prepared in Example 1-1 above, was treated with SmaI, a restriction enzyme, followed by DNA purification. Thereafter, a truncated linear cloning vector was prepared. The gene, *pntAB,* was amplified by PCR using *W3110* genomic DNA as a template and SEQ ID NOS: 8 and 9 as primers. Specifically, denaturation was performed at 94°C for 30 seconds, annealing at 55°C for 30 seconds, and extension at 72°C for 3 minutes using SolGent™ Pfu-X DNA Polymerase (SolGent, DNA); and this was repeated 30 times.

Additionally, the gene, *asd,* was amplified as described above; that is, PCR was performed using *W3110* genomic DNA as a template and SEQ ID NOS: 10 and 11 as primers. In addition, the gene, *aspC,* was amplified by PCR using *W3110* genomic DNA as a template and SEQ ID NOS: 12 and 13 as primers.

The threonine operon (thrO) was amplified from genomic DNA of *KCCM10541* (Korean Patent No. 10-0576342) by PCR using SEQ ID NOS: 14 and 15 as primers. Each of the four DNAs and vector DNAs prepared in the above underwent a Gibson assembly method to prepare the plasmid, *pMu-R6K_pntAB asd aspC-thrO* (Daniel G Gibson, et al., Nature Methods 2009).

In the Gibson assembly method, 5X ISO buffer was prepared using the composition shown in Table 1 below, and then the Gibson assembly master mix (15 µL), in which the 5X ISO buffer was mixed with the three enzymes (e.g., T5 exonuclease (Epicentre), Phusion polymerase (New England Biolabs), and Taq ligase (New England Biolabs)) using the composition shown in Table 2 below, was reacted with the DNAs (5 µL) to be cloned at 50°C for 1 hour. Thereafter, electroporation (2500 V) was applied to competent cells of TransforMaxTM EC100DTM pir-116 Electrocompetent *E. coli* (Epicentre, USA). The strains were recovered therefrom, plated on LB plate medium containing ampicillin (100 µg/L), and then cultured overnight at 37°C. Thereafter, the strains showing resistance to ampicillin were selected. A plasmid was obtained from the selected strains, and whether it had a desired nucleotide sequence was determined by sequencing analysis.

**[Table 1]**

| Composition of 5X ISO buffer | |
|---|---|
| Component | Content |
| 1M Tris-HCl pH 7.5 | 3 mL |
| 2 M MgCl₂ | 150 µL |
| 100 nM dNTP mix | 240 µL |
| 1 M DTT | 300 µL |
| PEG-8000 | 1.5 g |
| 100 mM NAD | 300 µL |
| Total | dH₂O up to 6 mL |

| | |
|---|---|
| dH₂O: distilled water. | |

**[Table 2]**

| Composition of Gibson assembly master mix | |
|---|---|
| Component | Content |
| 5X ISO buffer | 320 µL |
| 10 U/µLL T5 exonuclease | 0.64 µL |
| 2 U/µL Phusion polymerase | 20 µL |
| 40 U/µL Taq ligase | 160 µL |
| Total | dH₂O up to 1.2 mL |

### 1-2-2: pMu-R6K gdhA aspC thrO

*pMu-R6K,* the integrative plasmid prepared in Example 1-1, was treated with SmaI, a restriction enzyme, in the same manner as in Example 1-2-1, followed by DNA purification. Thereafter, a truncated linear cloning vector was prepared. From *W3110* genomic DNA, the gene, *gdhA,* was amplified by PCR using SEQ ID NOS: 18 and 19 as primers, and the gene,

*aspC,* was amplified by PCR using SEQ ID NOS: 20 and 21 as primers. The threonine operon (thrO) was amplified from KCCM10541 genomic DNA by PCR using SEQ ID NOS: 14 and 15 as primers. Each of the three DNAs and vectors DNAs prepared in the above underwent the Gibson assembly method to prepare the plasmid, *pMu-R6K_gdhA aspC thrO.*

### 1-2-3: pMu-ppc aspA thrO

*pMu-R6K,* the integrative plasmid prepared in Example 1-1 above, was treated with SmaI, a restriction enzyme, in the same manner as in Example 1-2-1, followed by DNA purification. Thereafter, a truncated linear cloning vector was prepared. From *W3110* genomic DNA, the gene, *ppc,* was amplified by PCR using SEQ ID NOS: 22 and 23 as primers, and the gene, *aspA,* was amplified by PCR using SEQ ID NOS: 24 and 25 as primers. The threonine operon (thrO) was amplified from KCCM10541 genomic DNA by PCR using SEQ ID NOS: 26 and 27 as primers. Each of the three DNAs and vector DNAs prepared in the above underwent the Gibson assembly method to prepare the plasmid, *pMu-R6K_ppc aspA thrO.*

### 1-3. Preparation of threonine biosynthesis-enhanced wild-type E. coli

Wild-type *E. coli W3110,* in which *pCJ-MuAB* prepared in Example 1-1 above was transformed, was cultured at 30°C until OD₆₀₀ reached 0.6 using LB medium supplemented with ampicillin (100 µg/L) and 5 mM arabinose. Thereafter, the resultants were washed once with sterile distilled water and twice with 10% glycerol through a centrifuge to prepare competent cells (Sambrook and Russell 2001). The integrative plasmid, *pMu-R6K_pntAB asd aspC-thrO,* was electroporated with the prepared competent cells, SOC medium (1 mL) was added thereto, and then the resultants were incubated at 37°C for 1 hour. Thereafter, the resultants were plated on LB plate medium containing chloramphenicol (25 µg/L), and then colony PCR of chloramphenicol-resistant bacterial colonies was carried out using SEQ ID NOS: 10 and 15 to confirm that the gene, *pntAB asd aspC thrO,* was introduced into the genome. Thereafter, the plasmid *pJW168* was transformed into the selected strain, and then plated on LB plate medium supplemented with 0.1 mM IPTG to remove a chloramphenicol-resistant marker (*cat*) (Beatri'z Palmeros et al., 2000, Gene). The introduction of *pMu-R6K_gdhA aspC thrO* was sequentially carried out by the mini-Mu integration method as described above, and then colony PCR was performed using SEQ ID NOS: 18 and 15 to confirm whether the introduction was successful. In addition, after the introduction of *pMu-ppc aspA thrO,* colony PCR was performed using SEQ ID NOS: 22 and 27 to confirm such introduction. The thus-produced wild-type *E. coli* (*W3110*)-based threonine-producing strain was named *CJT1.*

### Example 2: Preparation of ilvA gene-deficient strain

An FRT-one-step-PCR deletion method was used to delete the gene *ilvA,* which is involved in a representative pathway for threonine degradation (Kirill A, et al., 2000, PNAS). A deletion cassette was prepared by PCR using the primers of SEQ ID NOS: 28 and 29 with the vector *pKD3* as a template. Denaturation was performed at 94°C for 30 seconds, annealing at 55°C for 30 seconds, and extension at 72°C for 3 minutes using SolGent™ Pfu-X DNA Polymerase (SolGent, Korea); and this was repeated 30 times.

The resulting PCR product was electrophoresed on a 1.0% agarose gel, and the *ilvA*-deficient cassette was purified from a band having a size of 1.2 kbp. *KCCM10541 pKD46* and *CJT1 pKD46,* the strains in which the vector *pKD46* (Kirill A, et al., 2000, PNAS) was introduced, were cultured on LB medium containing ampicillin (100 µg/L) and 5 mM L-arabinose at 30°C/200 rpm until OD₆₀₀ reached 0.6. Thereafter, the resultants were washed once with sterile distilled water and twice with 10% glycerol to prepare competent cells. The *ilvA*-deficient cassette was introduced into the prepared competent cells through electroporation (2500 V). Thereafter, SOC medium (1 mL) was added thereto, and then the resultants were cultured overnight at 37°C. The strains showing the resistance were selected.

The selected strains were subjected to colony PCR using the primers of SEQ ID NOS: 28 and 29, and the *ilvA* gene deficiency was identified by observing that the size of the gene on the 1.0% agarose gel was 1.2 kb. The identified strains were once again transformed into the vector *pCP20* (Kirill A, et al., 2000, PNAS), and the final *ilvA* gene-deficient strain, which had a gene size of 150 bp on the 1.0% agarose gel, was prepared through colony PCR of the colonies, which were obtained by plating the transformed strains on LB plate medium containing ampicillin (100 µg/L), under the same conditions. The strain in which *ilvA* is deleted from *KCCM10541* was named *KCCM10541-ilvA*; and the strain in which *ilvA* is deleted from *CJT1* was named *CJT1-ilva.*

### Example 3: Preparation of cimA gene-introduced strain

DNAs, *Pcj1_cimA, Pcj1_cimA 2.0,* and *Pcj1_cimA* 3.7 (Atsumi, Liao JC, Appl Environ Microbiol. 2008), were prepared by codon-optimization based on the gene *cimA* derived from *Methanococcus jannaschii* containing the CJ1 promoter (Korean Patent No. 10-0620092). A BamHI site was added to both ends when synthesizing the gene, and then subcloned into the CopyControl pCC1BAC (BamHI) (Epicentre, USA) to prepare the plasmids, *pCC1BAC: Pcj1_cimA, pCC1BAC: Pcj1_cimA 2.0,* and *pCC1BAC: Pcj1_cimA* 3.7 which finally express the gene *cimA.* In addition, the three plasmids above and the control plasmid *pCC1BAC* were transformed into the strains, *KCCM10541-ilvA* and *CJT1-ilvA,* by electroporation (2500 V) to finally prepare strains to be used for the experiments. For the comparison of the experiments, the strains in which *pCC1BAC* was transformed into the two strains, *KCCM10541* and *CJT1,* in the same manner as described above was also prepared.

In the above, the gene *cimA* encoded wild-type citramalate synthase; and *cimA 2.0* and *cimA* 3.7 encoded mutant-type citramalate synthase in which specific activities were increased and in which feedback-inhibition by isoleucine was reduced (Atsumi, Liao JC, Appl Environ Microbiol. 2008). The amino acid sequences for *cimA 2.0* and *cimA* 3.7 were SEQ ID NOS: 2 and 3, and the nucleotide sequences corresponding thereto are SEQ ID NOS: 5 and 6, respectively.

Recombinant microorganisms of *KCCM10541 pCC1BAC, KCCM10541-ilvA pCC1BAC, KCCM10541-ilvA pCC1BAC:Pcj1_cimA, KCCM10541-ilvA pCC1BAC:Pcj1_cimA 2.0,* and *KCCM10541-ilvA pCC1BAC:Pcj1_cimA* 3.7 were prepared based on *KCCM10541* or *KCCM10541-ilvA.* In addition, recombinant microorganisms of *CJT1 pCC1BAC, CJT1-ilvA pCC1BAC, CJT1-ilvA pCC1BAC:Pcj1_cimA, CJT1-ilvA pCC1BAC:Pcj1_cimA 2.0,* and *CJT1-ilvA pCC1BAC:Pcj1_cimA* 3.7 were prepared based on *CJT1* or *CJT1-ilvA.*

The present inventors named the recombinant microorganisms of *'CJT1-ilvA pCC1BAC:Pcj1_cimA', 'CJT1-ilvA pCC1BAC:Pcj1_cimA 2.0',* and *'CJT1-ilvA pCC1BAC:Pcj1_cimA 3.7'* as *'CA03-8303', 'CA03-8304',* and *'CA03-8305',* respectively. In addition, these microorganisms were deposited under the Budapest Treaty to the Korean Culture Center of Microorganisms (KCCM) on December 5, 2014, and the microorganisms were designated as 'KCCM11632P', 'KCCM11633P', and 'KCCM11634P', respectively.

### Example 4: Confirmation of threonine productivity by flask evaluation of ilvA-deficient and cimA-expressing strains

Flask evaluation was performed in order to compare the amount of threonine produced in the strains prepared in Example 3 above. In the flask test, each of the strains was streaked on a LB plate containing chloramphenicol (25 µg/L) and cultured in an incubator (33°C) for 16 hours. Thereafter, a single colony was inoculated in LB medium (2 mL), and the resultants were cultured in an incubator (200 rpm/33°C) for 12 hours. In addition, the threonine-producing flask medium (25 mL) shown in Table 3 below was added to a flask (250 mL), and 500 µL of the medium solution previously cultured was added thereto. Thereafter, the flask was incubated in an incubator (200 rpm/33°C) for 48 hours, and then the amount of threonine obtained from each strain was compared. The results are shown in Table 4 below.

**[Table 3]**

| Composition of threonine-producing flask medium | |
|---|---|
| Composite | Concentration (per Liter) |
| Glucose | 70 g |
| Ammonium Sulfate | 25 g |
| KH₂PO₄ | 1 g |
| MgSO₄·7H₂O | 0.5 g |
| FeSO₄·7H₂O | 5 mg |
| MnSO₄· 8H₂O | 5 mg |
| ZnSO₄ | 5 mg |
| Calcium carbonate | 30 g |
| Yeast extract | 2 g |
| Methionine | 0.3 g |
| pH | 6.8 |

**[Table 4]**

| Production of threonine in the producing-strain by flask culture | | | | |
|---|---|---|---|---|
| Strain | OD | Amount of sugar consumed (g/L) | Amount of threonine produced (g/L) | Amount of Acetate produced (g/L) |
| *KCCM10541* *pCC1BAC* | 21.6 | 70 | 34.7 | 0.35 |
| *KCCM10541-ilvA* *pCC1BAC* | 5.5 | 12.0 | 6.6 | 0.11 |
| *KCCM10541-ilvA* *pCC1 BAC:Pcj1_cimA* | 18.5 | 70 | 37.5 | 0.15 |
| *KCCM10541-ilvA* *pCC1BAC:Pcj1_cimA 2.0* | 22.5 | 70 | 38.3 | 0.10 |
| *KCCM10541-ilvA* *pCC1BAC:Pcj1_cimA* 3.7 | 23.2 | 70 | 37.8 | 0.08 |
| *CJT1* *pCC1BAC* | 35.2 | 70 | 11.4 | 2.15 |
| *CJT1-ilvA* *pCC1BAC* | 6.4 | 13.6 | 3.0 | 1.24 |
| *CJT1-ilvA* *pCC1 BAC:Pcj1_cimA* | 30.6 | 70 | 14.3 | 1.42 |
| *CJT1-ilvA* *pCC1BAC:Pcj1_cimA 2.0* | 36.0 | 70 | 14.7 | 1.32 |
| *CJT1-ilvA* *pCC1BAC:Pcj1_cimA* 3.7 | 37.0 | 70 | 14.5 | 1.34 |

As shown in Table 4 above, it was observed that the two strains *KCCM10541-ilvA pCC1BAC* and *CJT1-ilvA pCC1BAC,* in which *ilvA* was deleted, did not grow well due to the requirement of isoleucine compared to strains *KCCM10541 pCC1BAC* and *CJT1 pCC1BAC,* in which *ilvA* was not deleted (see OD values). However, it was confirmed that in *KCCM10541-ilvA pCC1BAC:Pcj1_cimA, KCCM10541-ilvA pCC1BAC:Pcj1_cimA 2.0, KCCM10541-ilvA pCC1BAC:Pcjl_cimA 3.7, CJT1-ilvA pCC1BAC:Pcj1_cimA, CJT1-ilvA pCC1BAC:Pcj1_cimA 2.0,* and *CJT1-ilvA pCC1BAC:Pcj1_cimA* 3.7, which are the variant strains in which three types of *cimA* were introduced, the growth was completely recovered and the amount of threonine produced was increased by about 10% to 30%, while the amount of acetate produced was reduced by about 30% to 60%.

In particular, it was confirmed that when *cimA 2.0* or *cimA* 3.7 was introduced, the strain growth ability was increased by 20% or higher and the amount of threonine produced was slightly increased, while the amount of acetate produced was further reduced, compared to the case in which *cimA* was introduced into the *ilvA*-deficient strains.

From the foregoing, one of ordinary skill in the art to which the present disclosure pertains will be able to understand that the present disclosure may be embodied in other specific forms without modifying the technical concepts or essential characteristics of the present disclosure. In this regard, the exemplary embodiments disclosed herein are only for illustrative purposes and should not be construed as limiting the scope of the present disclosure. On the contrary, the present disclosure is intended to cover not only the exemplary embodiments but also various alternatives, modifications, equivalents, and other embodiments that may be included within the spirit and scope of the present disclosure as defined by the appended claims.

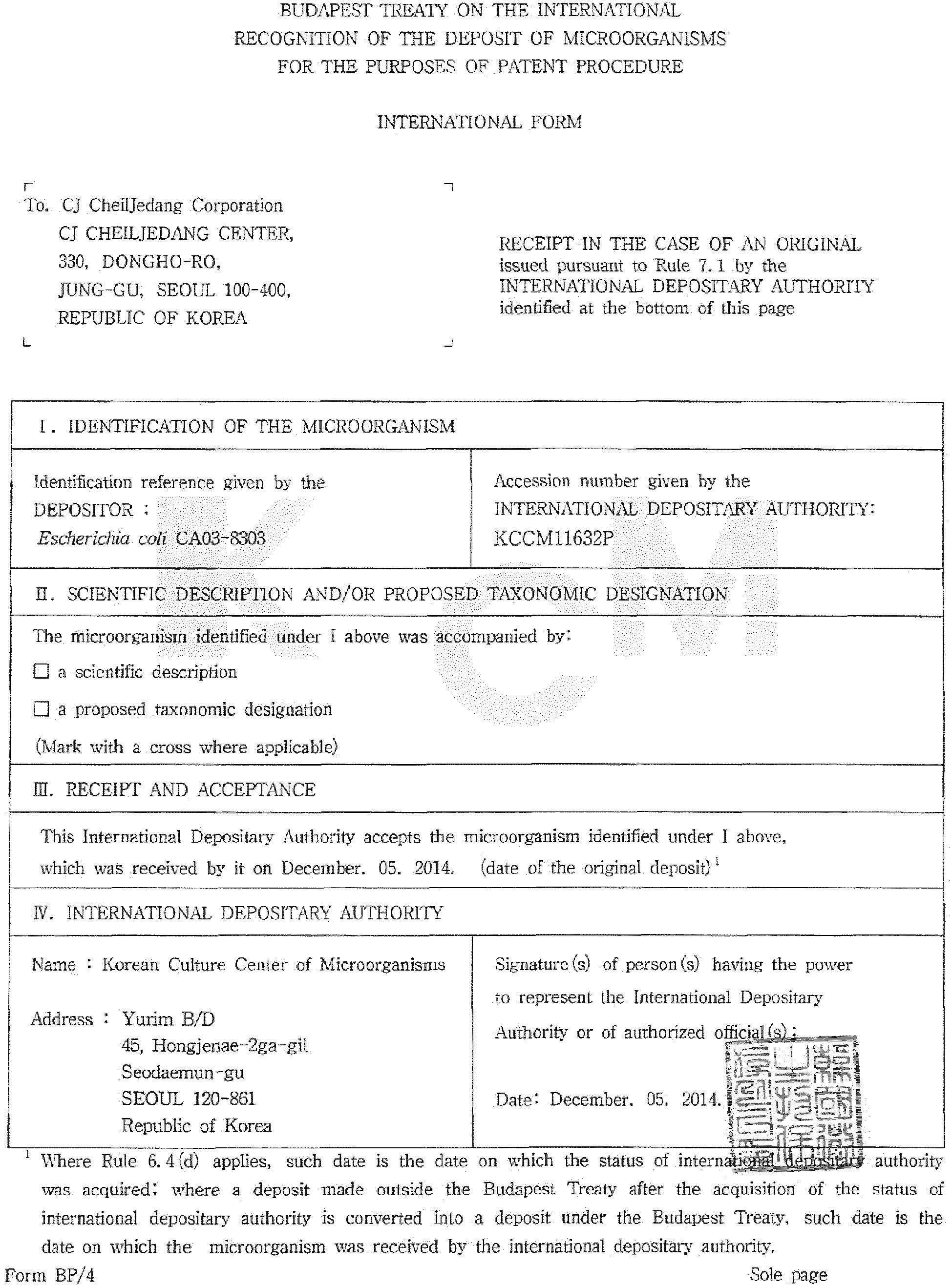
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400,
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR :
Escherichia coli CA03-8303 | Accession number given by the
INTERNATIONAL DEPOSITARY AUTHORITY:
KCCM11632P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 05. 2014. (date of the original deposit)¹
IV. INTERNATIONAL DEPOSITARY AUTHORITY
Name : Korean Culture Center of Microorganisms

Address : Yurim B/D
45, Hongjenae-2ga-gil
Seodaemun-gu
SEOUL 120-861
Republic of Korea | Signature(s) of person(s) having the power
to represent the International Depositary
Authority or of authorized official(s):

Date: December. 05. 2014.

¹ Where Rule 6.4(d) applies, such date is the date on which the status of international depositary authority
was acquired; where a deposit made outside the Budapest Treaty after the acquisition of the status of
international depositary authority is converted into a deposit under the Budapest Treaty, such date is the
date on which the microorganism was received by the international depositary authority.
Form BP/4 Sole page

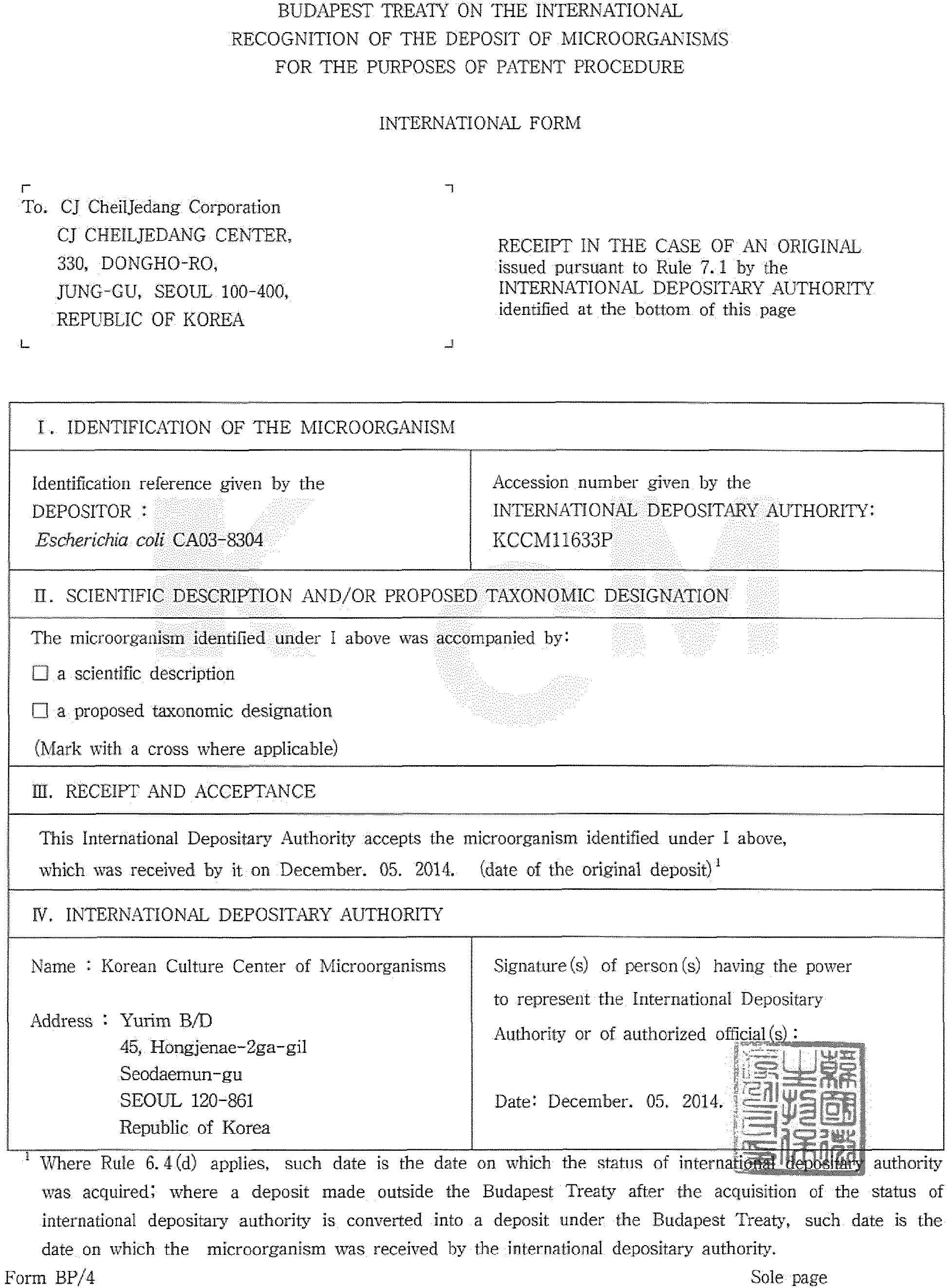
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400,
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR :
Escherichia coli CA03-8304 | Accession number given by the
INTERNATIONAL DEPOSITARY AUTHORITY:
KCCM11633P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 05. 2014. (date of the original deposit)¹
IV. INTERNATIONAL DEPOSITARY AUTHORITY
Name : Korean Culture Center of Microorganisms

Address : Yurim B/D
45, Hongjenae-2ga-gil
Seodaemun-gu
SEOUL 120-861
Republic of Korea | Signature(s) of person(s) having the power
to represent the International Depositary
Authority or of authorized official(s):
Date: December. 05. 2014.

¹ Where Rule 6.4(d) applies, such date is the date on which the status of international depositary authority
was acquired; where a deposit made outside the Budapest Treaty after the acquisition of the status of
international depositary authority is converted into a deposit under the Budapest Treaty, such date is the
date on which the microorganism was received by the international depositary authority.
Form BP/4 Sole page

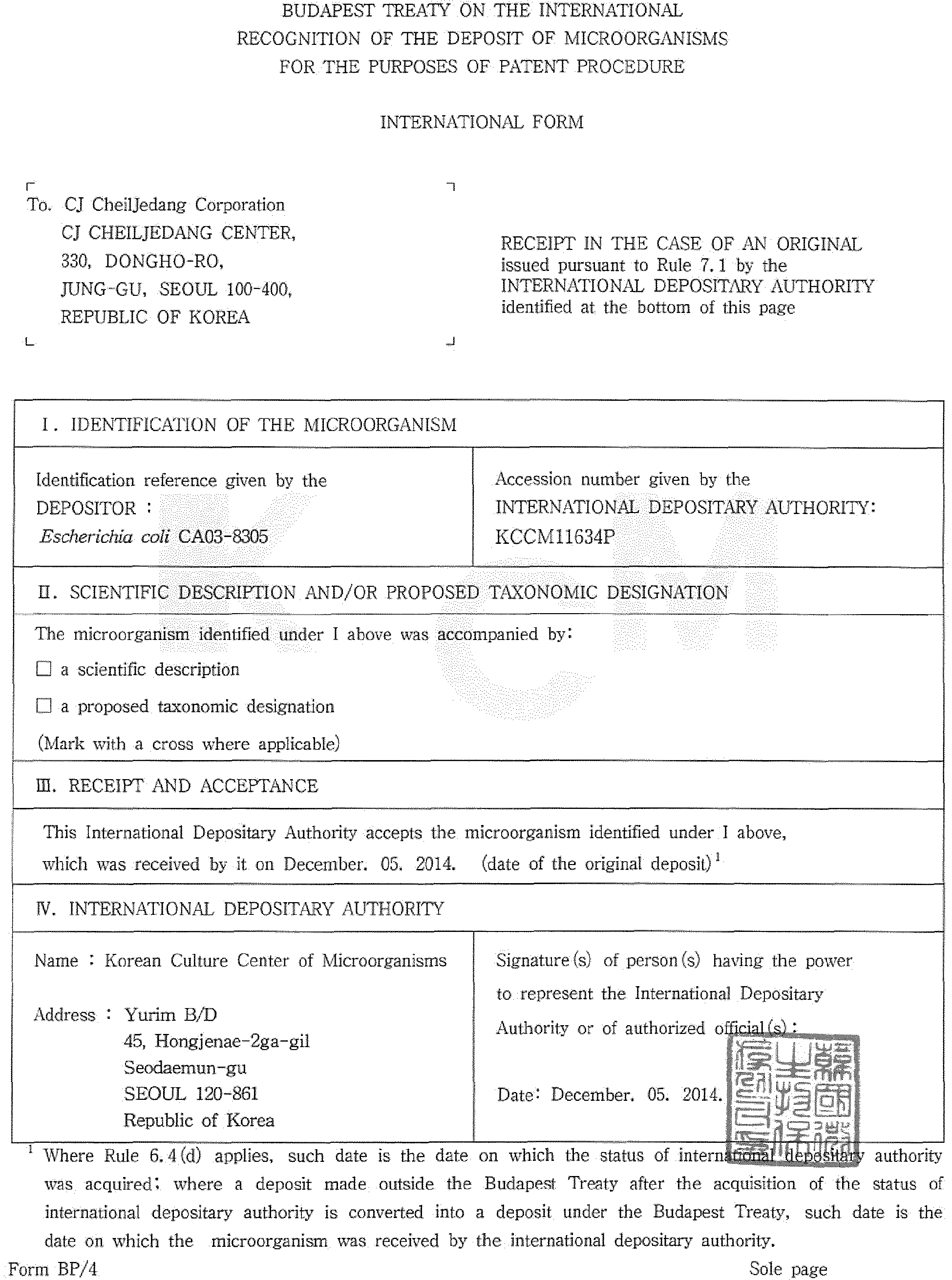
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400,
REPUBLIC OF KOREA

RECEIPT IN THE CASE OF AN ORIGINAL
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR :
Escherichia coli CA03-8305 | Accession number given by the
INTERNATIONAL DEPOSITARY AUTHORITY:
KCCM11634P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on December. 05. 2014. (date of the original deposit)¹
IV. INTERNATIONAL DEPOSITARY AUTHORITY
Name : Korean Culture Center of Microorganisms

Address : Yurim B/D
45, Hongjenae-2ga-gil
Seodaemun-gu
SEOUL 120-861
Republic of Korea | Signature(s) of person(s) having the power
to represent the International Depositary
Authority or of authorized official(s):
Date: December. 05. 2014.
¹ Where Rule 6.4(d) applies, such date is the date on which the status of international depositary authority
was acquired; where a deposit made outside the Budapest Treaty after the acquisition of the status of
international depositary authority is converted into a deposit under the Budapest Treaty, such date is the
date on which the microorganism was received by the international depositary authority.
Form BP/4 Sole page

<110>    CJ CheilJedang Corporation

<120>    Microorganisms having L-threonine productivity and a process for
         producing L-threonine using the same

<130>    OPA16176-PCT

<150>    KR 10-2015-0148004
<151>    2015-10-23

<160>    30

<170>    KoPatentIn 3.0

<210>    1
<211>    491
<212>    PRT
<213>    Methanococcus jannaschii

<400>    1
Met Met Val Arg Ile Phe Asp Thr Thr Leu Arg Asp Gly Glu Gln Thr
  1               5                  10                  15

Pro Gly Val Ser Leu Thr Pro Asn Asp Lys Leu Glu Ile Ala Lys Lys
             20                  25                  30

Leu Asp Glu Leu Gly Val Asp Val Ile Glu Ala Gly Ser Ala Ile Thr
         35                  40                  45

Ser Lys Gly Glu Arg Glu Gly Ile Lys Leu Ile Thr Lys Glu Gly Leu
     50                  55                  60

Asn Ala Glu Ile Cys Ser Phe Val Arg Ala Leu Pro Val Asp Ile Asp
 65                  70                  75                  80

Ala Ala Leu Glu Cys Asp Val Asp Ser Val His Leu Val Val Pro Thr
                 85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu His Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Thr Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile

225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys Leu Val Glu Glu Lys Ile Lys Leu Asp Glu Leu Thr Val
    370                 375                 380

Val Ser Gly Asn Lys Ile Thr Pro Ile Ala Ser Val Lys Leu His Tyr
385                 390                 395                 400

Lys Gly Glu Asp Ile Thr Leu Ile Glu Thr Ala Tyr Gly Val Gly Pro
                405                 410                 415

Val Asp Ala Ala Ile Asn Ala Val Arg Lys Ala Ile Ser Gly Val Ala
            420                 425                 430

Asp Ile Lys Leu Val Glu Tyr Arg Val Glu Ala Ile Gly Gly Gly Thr
        435                 440                 445

Asp Ala Leu Ile Glu Val Val Val Lys Leu Arg Lys Gly Thr Glu Ile
    450                 455                 460

Val Glu Val Arg Lys Ser Asp Ala Asp Ile Ile Arg Ala Ser Val Asp
465                 470                 475                 480

Ala Val Met Glu Gly Ile Asn Met Leu Leu Asn
                485                 490


<210>   2
<211>   371
<212>   PRT
<213>   Artificial Sequence

<220>
<223>   Methanococcus jannaschii cimA variant, cimA 2.0 a.a.


<400>   2
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Glu Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Leu Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys
    370

<210>   3
<211>   371
<212>   PRT
<213>   Artificial Sequence

<220>
<223>   Methanococcus jannaschii cimA variant, cimA 3.7 a.a.

<400>   3
Met Met Val Arg Ile Phe Asp Thr Thr Leu Arg Asp Gly Glu Gln Thr
1               5                   10                  15

Pro Gly Val Ser Leu Thr Pro Asn Asp Lys Leu Glu Ile Ala Lys Lys
            20                  25                  30

Leu Asp Glu Leu Gly Val Asp Val Ile Glu Ala Gly Ser Ala Val Thr
        35                  40                  45

Ser Lys Gly Glu Arg Glu Gly Ile Lys Leu Ile Thr Lys Glu Gly Leu
    50                  55                  60

Asn Ala Glu Ile Cys Ser Phe Val Arg Ala Leu Pro Val Asp Ile Asp
65                  70                  75                  80

Ala Ala Leu Glu Cys Asp Val Asp Ser Val His Leu Val Val Pro Thr
                85                  90                  95

Ser Pro Ile His Met Lys Tyr Lys Leu Arg Lys Thr Glu Asp Glu Val
            100                 105                 110

Leu Val Thr Ala Leu Lys Ala Val Glu Tyr Ala Lys Glu Gln Gly Leu
        115                 120                 125

Ile Val Glu Leu Ser Ala Glu Asp Ala Thr Arg Ser Asp Val Asn Phe
    130                 135                 140

Leu Ile Lys Leu Phe Asn Glu Gly Glu Lys Val Gly Ala Asp Arg Val
145                 150                 155                 160

Cys Val Cys Asp Thr Val Gly Val Leu Thr Pro Gln Lys Ser Gln Glu
                165                 170                 175

Leu Phe Lys Lys Ile Thr Glu Asn Val Asn Leu Pro Val Ser Val His
            180                 185                 190

Cys His Asn Asp Phe Gly Met Ala Thr Ala Asn Ala Cys Ser Ala Val
        195                 200                 205

Leu Gly Gly Ala Val Gln Cys His Val Thr Val Asn Gly Ile Gly Glu
    210                 215                 220

Arg Ala Gly Asn Ala Ser Leu Glu Glu Val Val Ala Ala Ser Lys Ile
225                 230                 235                 240

Leu Tyr Gly Tyr Asp Thr Lys Ile Lys Met Glu Lys Leu Tyr Glu Val
                245                 250                 255

Ser Arg Ile Val Ser Arg Leu Met Lys Leu Pro Val Pro Pro Asn Lys
            260                 265                 270

Ala Ile Val Gly Asp Asn Ala Phe Ala His Glu Ala Gly Ile His Val
        275                 280                 285

Asp Gly Leu Ile Lys Asn Thr Glu Thr Tyr Glu Pro Ile Lys Pro Glu
    290                 295                 300

Met Val Gly Asn Arg Arg Arg Ile Ile Leu Gly Lys His Ser Gly Arg
305                 310                 315                 320

Lys Ala Leu Lys Tyr Lys Leu Asp Leu Met Gly Ile Asn Val Ser Asp
                325                 330                 335

Glu Gln Leu Asn Lys Ile Tyr Glu Arg Val Lys Glu Phe Gly Asp Leu
            340                 345                 350

Gly Lys Tyr Ile Ser Asp Ala Asp Leu Leu Ala Ile Val Arg Glu Val
        355                 360                 365

Thr Gly Lys
    370

<210>    4
<211>    1476
<212>    DNA
<213>    Methanococcus jannaschii

<400>    4
atgatggtaa ggatattcga taccacactt cgcgacggag agcagacacc aggagtttct      60

ttaacaccaa acgataagct ggagatagct aaaaagttgg atgaacttgg ggttgatgtt     120

atagaggccg gtagcgctat cactagcaag ggagagcgcg aaggaataaa actcattaca     180

aaagaaggtc ttaacgcaga aatctgcagt ttcgtgcgtg cactccctgt agacatagac     240

gctgccttag aatgtgatgt agatagtgtg cacctcgtgg tgcccacatc tccaatacac     300

atgaagtaca aacttcgtaa aacggaggat gaggttctcg agaccgcact taaggctgtg     360

gaatacgcta aagagcatgg cttgatcgtt gagttatcag cggaggatgc gacaagatcc     420

gatgttaatt tcttaataaa actcttcaat gaaggggaaa aggttggagc agaccgtgtt     480

tgtgtttgcg acaccgtagg agttttaacg ccacaaaagt cgcaggaact gtttaagaaa     540

ataaccgaga atgtgaattt accggtctcg gttcattgcc acaacgactt tggaatggct     600

actgctaata cttgctccgc agtgctcggc ggagctgttc agtgccacgt gacagttaac     660

ggtattggcg agcgcgcagg gaatgcctcc ttagaagagg tcgttgccgc gctgaaaatc     720

ctctacggct atgatactaa aataaagatg gaaaagttat acgaggtttc acgcattgtc     780

tcccgcttga tgaaacttcc tgttcccccg aacaaagcga ttgttggcga caatgcgttc     840

gctcacgaag caggtatcca tgtcgatgga ttaattaaaa atactgaaac ctatgagcca     900

atcaaaccag aaatggtggg caaccgccgg cgtatcattc tcggtaagca ctctggtcgg 960

aaagcgttaa agtacaagct tgatctaatg ggcataaacg tgtctgatga gcaattaaac 1020

aaaatttatg aacgcgttaa agaattcggg gacctaggta agtacatttc agatgctgat 1080

ttgctcgcta tagttcgcga agtcacggga aagttggttg aggagaagat caaactggac 1140

gaactcacgg tggtaagcgg aaacaagata accccaattg catctgttaa gctccattac 1200

aaaggagagg atatcacttt aatagagact gcttacggtg ttggaccggt cgatgcagca 1260

attaacgccg tgcgtaaagc aatatcggga gttgcagaca ttaagctcgt agagtatcgc 1320

gttgaagcga ttggtggcgg tactgatgcg ttaatagagg ttgtagttaa attacgtaaa 1380

gggactgaaa tagttgaggt tcgcaagtca gacgcggaca taatacgcgc ttctgtggac 1440

gctgtaatgg aaggaatcaa catgttgttg aattaa 1476

<210> 5
<211> 1116
<212> DNA
<213> Artificial Sequence

<220>
<223> Methanococcus jannaschii cimA variant, cimA 2.0

<400> 5
atgatggtaa ggatattcga taccacactt cgcgacggag agcagacacc aggagtttct 60

ttaacaccaa acgataagct ggagatagct aaaaagttgg atgaacttgg ggttgatgtt 120

atagaggccg gtagcgctgt cactagcaag ggagagcgcg aaggaataaa actcattaca 180

aaagaaggtc ttaacgcaga aatctgcagt ttcgtgcgtg cactccctgt agacatagac 240

gctgccttag aatgtgatgt agatagtgtg cacctcgtgg tgcccacatc tccaatacac 300

atgaagtaca aacttcgtaa aacggaggat gaggttctcg agaccgcact taaggctgtg 360

gaatacgcta aagagcaggg cttgatcgtt gagttatcag cggaggatgc gacaagatcc 420

gatgttaatt tcttaataaa actcttcaat gaaggggaaa aggttggagc agaccgtgtt 480

tgtgtttgcg acaccgtagg agttttaacg ccacaaaagt cgcaggaact gtttaagaaa 540

ataaccgaga atgtgaattt accggtctcg gttcattgcc acaacgactt tggaatggct 600

actgctaatg cttgctccgc agtgctcggc ggagctgttc agtgccacgt gacagttaac 660

ggtattggcg agcgcgcagg gaatgcctcc ttagaagagg tcgttgccgc gctgaaaatc 720

ctctacggct atgatactaa aataaagatg gaaaagttat acgaggtttc acgcattgtc 780

tcccgcttga tgaaacttcc tgttcccccg aacaaagcga ttgttggcga caatgcgttc 840

gctcacgaag caggtatcca tgtcgatgga ttaattaaaa atactgaaac ctatgagcca 900

atcaaaccag aaatggtggg caaccgccgg cgtatcattc tcggtaagca ctctggtcgg 960

aaagcgttaa agtacaagct tgatctaatg ggcataaacg tgtctgatga gcaattaaac 1020

aaaatttatg aacgcgttaa agaattcggg gacctaggta agtacatttc agatgctgat 1080

ttgctcgcta tagttcgcga agtcacggga aagtag 1116

<210> 6
<211> 1116
<212> DNA
<213> Artificial Sequence

<220>
<223> Methanococcus jannaschii cimA variant, cimA 3.7

<400> 6
atgatggtaa ggatattcga taccacactt cgcgacggag agcagacacc aggagtttct 60

ttaacaccaa acgataagct ggagatagct aaaaagttgg atgaacttgg ggttgatgtt 120

atagaggccg gtagcgctgt cactagcaag ggagagcgcg aaggaataaa actcattaca 180

aaagaaggtc ttaacgcaga aatctgcagt ttcgtgcgtg cactccctgt agacatagac 240

gctgccttag aatgtgatgt agatagtgtg cacctcgtgg tgcccacatc tccaatacac 300

atgaagtaca aacttcgtaa aacggaggat gaggttctcg tgaccgcact taaggctgtg 360

gaatacgcta aagagcaggg cttgatcgtt gagttatcag cggaggatgc gacaagatcc 420

gatgttaatt tcttaataaa actcttcaat gaaggggaaa aggttggagc agaccgtgtt 480

tgtgtttgcg acaccgtagg agttttaacg ccacaaaagt cgcaggaact gtttaagaaa 540

ataaccgaga atgtgaattt accggtctcg gttcattgcc acaacgactt tggaatggct 600

actgctaatg cttgctccgc agtgctcggc ggagctgttc agtgccacgt gacagttaac 660

ggtattggcg agcgcgcagg gaatgcctcc ttagaagagg tcgttgccgc gtcgaaaatc 720

ctctacggct atgatactaa aataaagatg gaaaagttat acgaggtttc acgcattgtc 780

tcccgcttga tgaaacttcc tgttcccccg aacaaagcga ttgttggcga caatgcgttc 840

gctcacgaag caggtatcca tgtcgatgga ttaattaaaa atactgaaac ctatgagcca 900

atcaaaccag aaatggtggg caaccgccgg cgtatcattc tcggtaagca ctctggtcgg 960

aaagcgttaa agtacaagct tgatctaatg ggcataaacg tgtctgatga gcaattaaac 1020

aaaatttatg aacgcgttaa agaattcggg gacctaggta agtacatttc agatgctgat 1080

ttgctcgcta tagttcgcga agtcacggga aagtag 1116

<210> 7
<211> 514
<212> PRT
<213> Escherichia coli

<400> 7

<210>    8
<211>    40
<212>    DNA
<213>    Artificial Sequence

<220>
<223>    pMu-pntAB primer F

<400>    8
gtcgactcta gaggatcccc aagtagtgat tcgtgccggg                              40

<210>    9
<211>    40
<212>    DNA
<213>    Artificial Sequence

<220>
<223>    pMu-pntAB primer R

<400>    9
cccggcacga atcactactt ggggatcctc tagagtcgac                              40

<210>	10
<211>	40
<212>	DNA
<213>	Artificial Sequence

<220>
<223>	pntAB-asd primer F

<400>	10
gcgcaatttt gacaggatct tgattgcccg ccgtcttttc	40

<210>	11
<211>	40
<212>	DNA
<213>	Artificial Sequence

<220>
<223>	pntAB-asd primer R

<400>	11
gaaaagacgg cgggcaatca agatcctgtc aaaattgcgc	40

<210>	12
<211>	40
<212>	DNA
<213>	Artificial Sequence

<220>
<223>	asd-aspC primer F

<400>	12
gagttaaata gagcaagctc gtccacctat gttgactaca	40

<210>	13
<211>	40
<212>	DNA
<213>	Artificial Sequence

<220>
<223>	asd-aspC primer R

<400>	13
tgtagtcaac ataggtggac gagcttgctc tatttaactc	40

<210>	14
<211>	40
<212>	DNA
<213>	Artificial Sequence

<220>
<223>	aspC-thrO primer F

<400>	14
gcctgatgcg ctacgcttat agcttttcat tctgactgca	40

<210>	15
<211>	40
<212>	DNA
<213>	Artificial Sequence

<220>
<223>	aspC-thrO primer R


<400>	15
tgcagtcaga atgaaaagct ataagcgtag cgcatcaggc	40


<210>	16
<211>	40
<212>	DNA
<213>	Artificial Sequence

<220>
<223>	thrO-pMu primer F


<400>	16
tgatgatgaa tcatcagtaa gggtaccgag ctcgctgttt	40


<210>	17
<211>	40
<212>	DNA
<213>	Artificial Sequence

<220>
<223>	thrO-pMu primer R


<400>	17
aaacagcgag ctcggtaccc ttactgatga ttcatcatca	40


<210>	18
<211>	40
<212>	DNA
<213>	Artificial Sequence

<220>
<223>	pMu-gdhA primer F


<400>	18
gtcgactcta gaggatcccc gctactgata acggtagccg	40


<210>	19
<211>	40
<212>	DNA
<213>	Artificial Sequence

<220>
<223>	pMu-gdhA primer R

<400>    19
cggctaccgt tatcagtagc ggggatcctc tagagtcgac                              40


<210>    20
<211>    41
<212>    DNA
<213>    Artificial Sequence

<220>
<223>    gdhA-aspC primer F


<400>    20
tacgctctaa tgtaggccgg gtccacctat gttgactaca t                            41


<210>    21
<211>    41
<212>    DNA
<213>    Artificial Sequence

<220>
<223>    gdhA-aspC primer R


<400>    21
atgtagtcaa cataggtgga cccggcctac attagagcgt a                            41


<210>    22
<211>    40
<212>    DNA
<213>    Artificial Sequence

<220>
<223>    pMu-ppc primer F


<400>    22
gtcgactcta gaggatcccc gtaggccgga taaggcgctc                              40


<210>    23
<211>    40
<212>    DNA
<213>    Artificial Sequence

<220>
<223>    pMu-ppc primer R


<400>    23
gagcgcctta tccggcctac ggggatcctc tagagtcgac                              40


<210>    24
<211>    40
<212>    DNA
<213>    Artificial Sequence

<220>
<223>    ppc-aspA primer F

<400>    24
ctgtctgcat tttattcaaa ctacctgaat gggttgcgaa                              40


<210>    25
<211>    40
<212>    DNA
<213>    Artificial Sequence

<220>
<223>    ppc-aspA primer R


<400>    25
ttcgcaaccc attcaggtag tttgaataaa atgcagacag                              40


<210>    26
<211>    40
<212>    DNA
<213>    Artificial Sequence

<220>
<223>    aspA-thrO primer F


<400>    26
taaaaaacaa ggaaggctaa agcttttcat tctgactgca                              40


<210>    27
<211>    40
<212>    DNA
<213>    Artificial Sequence

<220>
<223>    aspA-thrO primer R


<400>    27
tgcagtcaga atgaaaagct ttagccttcc ttgtttttta                              40


<210>    28
<211>    86
<212>    DNA
<213>    Artificial Sequence

<220>
<223>    primer for ilvA deficiency F


<400>    28
gcctgacgga agaacagaaa gcgcacggcg tgatcactgc ttctgcgggt aaccacgcgc        60

agggcgctgg agctgcttcg aagttc                                             86


<210>    29
<211>    86
<212>    DNA

<213>    Artificial Sequence

<220>
<223>    primer for ilvA deficiency R


<400>    29
gatattcata tggaccatgg ccggaaccct ctggcgcgct ggcgctggcg ggaatgaaaa    60

aatatatcgc cctgcacaac attcgc                                          86


<210>    30
<211>    1545
<212>    DNA
<213>    Escherichia coli

<400>    30
atggctgact cgcaacccct gtccggtgct ccggaaggtg ccgaatattt aagagcagtg    60

ctgcgcgcgc cggtttacga ggcggcgcag gttacgccgc tacaaaaaat ggaaaaactg    120

tcgtcgcgtc ttgataacgt cattctggtg aagcgcgaag atcgccagcc agtgcacagc    180

tttaagctgc gcggcgcata cgccatgatg gcgggcctga cggaagaaca gaaagcgcac    240

ggcgtgatca ctgcttctgc gggtaaccac gcgcagggcg tcgcgttttc ttctgcgcgg    300

ttaggcgtga aggccctgat cgttatgcca accgccaccg ccgacatcaa agtcgacgcg    360

gtgcgcggct tcggcggcga agtgctgctc cacggcgcga actttgatga agcgaaagcc    420

aaagcgatcg aactgtcaca gcagcagggg ttcacctggg tgccgccgtt cgaccatccg    480

atggtgattg ccgggcaagg cacgctggcg ctggaactgc tccagcagga cgcccatctc    540

gaccgcgtat ttgtgccagt cggcggcggc ggtctggctg ctggcgtggc ggtgctgatc    600

aaacaactga tgccgcaaat caaagtgatc gccgtagaag cggaagactc cgcctgcctg    660

aaagcagcgc tggatgcggg tcatccggtt gatctgccgc gcgtagggct atttgctgaa    720

ggcgtagcgg taaaacgcat cggtgacgaa accttccgtt tatgccagga gtatctcgac    780

gacatcatca ccgtcgatag cgatgcgatc tgtgcggcga tgaaggattt attcgaagat    840

gtgcgcgcgg tggcggaacc ctctggcgcg ctggcgctgg cgggaatgaa aaaatatatc    900

gccctgcaca acattcgcgg cgaacggctg gcgcatattc tttccggtgc caacgtgaac    960

ttccacggcc tgcgctacgt ctcagaacgc tgcgaactgg gcgaacagcg tgaagcgttg    1020

ttggcggtga ccattccgga agaaaaaggc agcttcctca aattctgcca actgcttggc    1080

gggcgttcgg tcaccgagtt caactaccgt tttgccgatg ccaaaaacgc ctgcatcttt    1140

gtcggtgtgc gcctgagccg cggcctcgaa gagcgcaaag aaattttgca gatgctcaac    1200

gacggcggct acagcgtggt tgatctctcc gacgacgaaa tggcgaagct acacgtgcgc    1260

tatatggtcg gcggacgtcc atcgcatccg ttgcaggaac gcctctacag cttcgaattc    1320

ccggaatcac cgggcgcgct gctgcgcttc ctcaacacgc tgggtacgta ctggaacatt    1380

tctttgttcc actatcgcag ccatggcacc gactacgggc gcgtactggc ggcgttcgaa 1440

cttggcgacc atgaaccgga tttcgaaacc cggctgaatg agctgggcta cgattgccac 1500

gacgaaacca ataacccggc gttcaggttc tttttggcgg gttag 1545

## Claims

1. A recombinant microorganism producing threonine, wherein an activity of threonine deaminase (IlvA) is reduced or inactivated and an activity of citramalate synthase is retained.

2. The recombinant microorganism of claim 1, wherein the threonine deaminase is represented by an amino acid sequence of SEQ ID NO: 7.

3. The recombinant microorganism of claim 1, wherein the citramalate synthase is citramalate synthase derived from *Methanococcus jannaschii.*

4. The recombinant microorganism of claim 1, wherein the citramalate synthase is represented by an amino acid sequence selected from the group consisting of SEQ ID NOS: 1, 2, and 3.

5. The recombinant microorganism of claim 1, wherein the recombinant microorganism is a microorganism of the genus *Corynebacterium* or a microorganism of the genus *Escherichia.*

6. The recombinant microorganism of claim 5, wherein the microorganism of the genus *Corynebacterium* is *Corynebacterium glutamicum* and the microorganism of the genus *Escherichia* is *Escherichia coli.*

7. A method for producing threonine, comprising:
culturing the recombinant microorganism of any one of claims 1 to 6 in a medium; and
recovering threonine from the microorganism or the cultured medium.
